# EUROPEAN PATENT SPECIFICATION

(11) **EP 1 894 579 B1**
(45) Date of publication and mention of the grant of the patent: **29.02.2012**
(21) Application number: 07253369.8
(22) Date of filing: 28.08.2007
(51) Int. Cl.: A61L 17/00, A61L 17/04, A61L 17/14

(54) **High strength suture coated with RGD peptide**
Mit RGD-Peptid beschichtete hochfeste Naht
Suture haute résistance revêtue de peptide RGD

(30) Priority: 28.08.2006 US 840467 P
(43) Date of publication of application: 05.03.2008
(73) Proprietor: Mazzocca, Augustus D., West Hartford, CT 06107 (US); McCarthy, Mary Beth, Kensington, CT 06037 (US)
(72) Inventor: Mazzocca, Augustus D., West Hartford, CT 06107 (US); McCarthy, Mary Beth, Kensington, CT 06037 (US)
(74) Representative: Oxley, Robin John George

(56) References cited:
- EP-A- 1 293 218
- US-A- 5 266 327
- US-A- 5 278 063

## Description

### FIELD OF THE INVENTION

The present invention relates to high strength surgical suture materials, and more particularly to braided suture blends of ultrahigh molecular weight polyethylene coated with RGD peptide.

### DESCRIPTION OF THE RELATED ART

Suture strength is an important consideration in any surgical suture material. One of the strongest materials currently formed into elongated strands is an ultrahigh molecular weight long chain polyethylene, typically used for fishing line and the like, which is sold under the trade names Dyneema or Spectra. This material is much stronger than ordinary surgical suture, however, it does not have acceptable knot tie down characteristics for use in surgical applications.

### SUMMARY OF THE INVENTION

A suture comprising a jacket comprising a plurality of braided yarns formed of ultrahigh molecular weight polyethylene, characterized in that the suture is coated with RGD peptide, the suture being coated by a process comprising the steps of
soaking a suture into a MES buffer;
removing the suture from the MES buffer, performing acid hydrolysis on the suture, and subsequently placing back the suture in the MES buffer;
removing the suture from the MES buffer and placing it in a MES buffer comprising EDC and sulfo-NHS; and
washing the suture with the MES buffer comprising EDC and sulfo-NHS and immersing the suture into a peptide solution to allow peptides to couple to the suture, to form the RGD coated suture.

Polyester fibers woven with the high molecular weight polyethylene provide improved tie down properties. RGD peptide promotes cell adhesion to substrates in vivo by interacting with receptors, integrins, on cell surface proteins. Further, RGD peptides also increase the permeability of endothelial monolayers and therefore may aid in surgical applications.

In a preferred embodiment, the suture includes a multifilament jacket formed of ultrahigh molecular weight polyethylene fibers braided with polyester. The jacket surrounds a fiber core made substantially or entirely of ultrahigh molecular weight polyethylene. The core preferably includes three strands of ultrahigh molecular weight polyethylene, twisted at about three to six twists per inch.

The jacket preferably comprises eight strands of ultrahigh molecular weight polyethylene braided with six strands of polyester. The tinted strands can be included in black or some other contrasting color.

Ultrahigh molecular weight polyethylene fibers suitable for use in the present invention are marketed under the Dyneema trademark by Toyo Boseki Kabushiki Kaisha, and are produced in the U.S. by Honeywell under the trademark Spectra.

The suture of the present invention advantageously has the strength of Ethibond No. 5 suture, yet has the diameter, feel and tie-ability of No. 2 suture. As a result, the suture of the present invention is ideal for most orthopedic procedures such as rotator cuff repair, Achilles tendon repair, patellar tendon repair, ACL/PCL reconstruction, hip and shoulder reconstruction procedures, and replacement for suture used in or with suture anchors.

The suture is coated with RGD peptide. RGD peptide suitable for use in the present invention is marketed under PCI-36xx-PI by Peptides International, and under GRADSP peptide and GRGDSP peptide by Calbiochem. Other products suitable for use in the present invention marketed by Pierce are: (i) Product #28390 - BupH MES Buffered Saline Packs to make a 500 ml of 0.1 M 2-[morpholino] ethanesulfonic acid, 0.9% NaCl, pH 4.7 when dissolved in 500 ml deionized water (5 liters total); (ii) Product # 22980 - -1-Ethyl-3-[3-dimethylaminopropyl]carbodiimide hydrochloride (EDC or EDAC), a zero-length crosslinking agent used to couple carboxyl groups to primary amines and a water-soluble carbodiimide for rapid preparation of peptide conjugates; (iii) Product #24510 - *N-*hydroxysulfosuccinimide (Sulfo-NHS), a product useful in improving the efficiency of EDC coupling.

A trace thread or two in the suture jacket aids surgeons in identifying the travel direction of the suture during surgery, particularly during operations viewed arthroscopically or remotely. Providing the trace threads in a regularly repeating pattern is particularly useful, allowing the surgeon to decode different ends of a length of suture, and to determine the direction of travel of a moving length of suture. The trace threads preferably are provided uniquely on each half of a length of suture to allow for tracing and identification of each end of the suture, such as when the suture is threaded through an eyelet of a suture anchor.

Other features and advantages of the present invention will become apparent from the following description of the invention which refers to the schematic accompanying drawings.

### BRIEF DESCRIPTION OF THE DRAWINGS

Fig. 1 is a copy of a scanning electron micrograph of a length of suture according to the present invention.

Fig. 2 is a schematic cross section of a length of suture according to the present invention.

Fig. 3 is an illustration of the suture of the present invention attached to a suture anchor loaded onto a driver.

Fig. 4A and 4B show the suture of the present invention attached to a half round, tapered needle.

### DETAILED DESCRIPTION OF THE INVENTION

The term "yarn(s)," as used herein, is to be understood as including fiber(s), filament(s), and the like used to make a suture of the present invention. Typically, though, yarns are comprised of fibers and/or filaments.

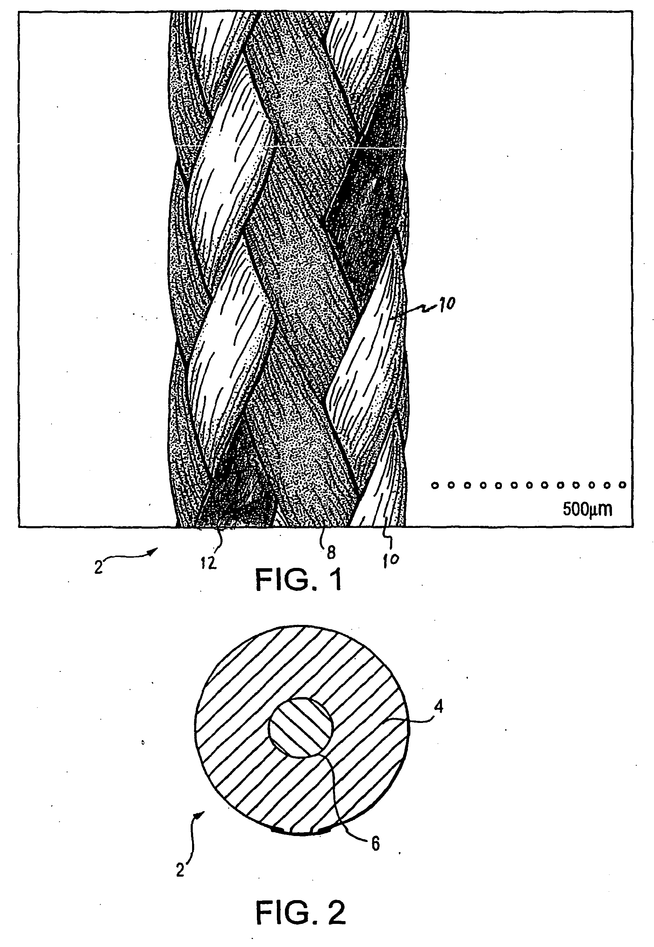

Referring to Fig. 1, a scanning electron micrograph of a length of suture 2 according to the present invention is shown. Suture 2 is made up of a jacket 4 and a core 6 surrounded by the jacket 4. See Fig. 2. Strands of ultrahigh molecular weight polyethylene (UHMWPE) 8, such as that sold under the tradenames Spectra and Dyneema, strands of polyester 10, and tinted strands 12 are braided together to form the jacket 4. Core 6 is formed of twisted strands of UHMWPE.

UHMWPE, used for strands 8, is substantially translucent or colorless. The polyester strands 10 are white (undyed). Due to the transparent nature of the UHMWPE, the suture takes on the color of strands 10 and 12, and thus appears to be white with a trace in the contrasting color.

In accordance with the present invention, trace strands 12 are preferably provided in black. The black trace assists surgeons in distinguishing between suture lengths with the trace and suture lengths without the trace. Traces also assist the surgeon in identifying whether the suture is moving. The trace can extend the entire length of the suture or only on half of a length of suture, the other half of the suture length remaining plain (white). Alternatively, the traces can form visibly distinct coding patterns on each half of the suture length. As a result, when the suture is threaded through the eyelet of a suture anchor, for example, the two legs (halves) of the length of suture are easily distinguished, and their direction of travel will be readily evident when the suture is pulled during surgery.

Details of the present invention will be described further below in connection with the following examples:

### Example: USP Size 5 (EP size 7)

Made on a 16 carrier Hobourns machine, the yarns used in the braided jacket are Honeywell Spectra 2000, polyester type 712, and nylon. The jacket is formed using eight strands of 144 decitex Spectra per carrier, braided with six strands of 100 decitex polyester, and two strands of tinted nylon. The core is formed of three carriers of 144 decitex Spectra braided at three to six twists per inch. A No. 5 suture is produced.

To make various sizes of the inventive suture, different decitex values and different PPI settings can be used to achieve the required size and strength needed. In addition, smaller sizes may require manufacture on 12 carrier machines, for example. The very smallest sizes can be made without a core. Overall, the suture may range from 5% to 90% ultrahigh molecular weight polymer (preferably at least 40% of the fibers are ultrahigh molecular weight polymer), with the balance formed of polyester and/or nylon. The core preferably comprises 18% or greater of the total amount of filament.

The suture is coated with RGD peptide (PCI-36xx-PI). The RGD peptide-coating increases permeability of endothelial monolayers, thereby resulting in a decrease in abrasion resistance to the suture. RGD peptides promote cell adhesion to substrates in vivo by interacting with receptors, integrins, on cell surface proteins and thus, RGD peptide-coated sutures perform better than other sutures in surgical applications.

Peptides may be coupled to or adsorbed to the suture. Typically, about 500 ml of coupling buffer, e.g., about 0.1M MES buffer, is prepared and a base, for example NaOH, is used to lower the acidity of the coupling buffer to a pH value of about 6.0.

In the present invention, peptides are coupled to a suture. For coupling peptides to a suture, the suture is preferably cut into 6 inch strips and soaked into an MES buffer for at least 30 minutes. Later, acid hydrolysis is performed, i.e., the suture is taken from the MES buffer and quickly dipped into an acid medium, for example, about 15 ml of 6N HCl and about 200 ul of H₂O₂. Thereafter, the suture is immediately put back into the MES buffer and washed several times. EDC and sulfo-NHS buffers are added to the MES buffer, for example, about 36mg of EDC and about 99mg of sulfo-NHS is added to about 90ml of MES buffer.

The suture is removed from the MES buffer and placed into the EDC buffer for about 15 minutes. The suture is then washed three times with the MES buffer. About 2 ml of peptide solution is immediately prepared and the suture immersed in the peptide solution. The peptide is allowed to react on the suture at 4°C. The suture is then washed about five times with MES buffer and then allowed to air dry under a hood.

The ultra high molecular weight (UHMW) polymer component of the present invention provides strength, and the polyester component is provided to improve tie ability and tie down characteristics. However, it has been found that the UHMW polymer provides an unexpected advantage of acting as a cushion for the polyester fibers, which are relatively hard and tend to damage each other. The UHMW polymer prevents breakage by reducing damage to the polyester when the suture is subjected to stress.

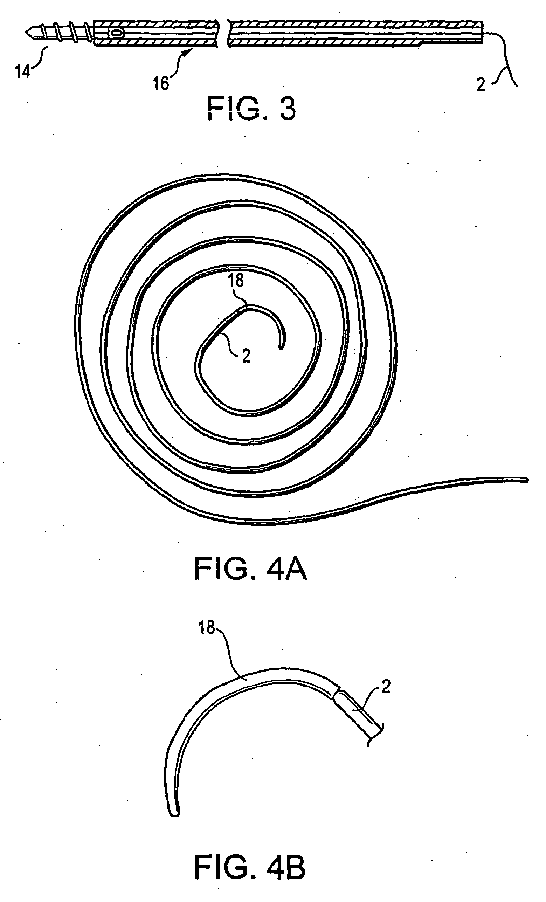

In one method of using the suture of the present invention, the suture 2 is attached to a suture anchor 14 as shown in Fig. 3 (prepackaged sterile with an inserter 16), or is attached at one or both ends to a half round, tapered needle 18 as shown in Figs. 4A and 4B. Fig. 4A also illustrates a length of suture having regularly repeating pattern of trace threads according to the present invention. Sections of the length of suture 2 have tinted tracing threads woven in. The alternating patterned and plain sections aid the surgeon in determining the direction of suture travel when pulling the suture, for example.

Although the present invention has been described in relation to particular embodiments thereof, many other variations and modifications and other uses will become apparent to those skilled in the art.

## Claims

1. A suture comprising a jacket comprising a plurality of braided yarns formed of ultrahigh molecular weight polyethylene, **characterized in that** the suture is coated with RGD peptide, the suture being coated by a process comprising the steps of:
soaking a suture into a MES buffer;
removing the suture from the MES buffer, performing acid hydrolysis on the suture, and subsequently placing back the suture in the MES buffer;
removing the suture from the MES buffer and placing it in a MES buffer comprising EDC and sulfo-NHS; and
washing the suture with the MES buffer comprising EDC and sulfo-NHS and immersing the suture into a peptide solution to allow peptides to couple to the suture, to form the RGD coated suture.

2. The suture of claim 1, further comprising a core comprising yarns of a ultrahigh molecular weight polyethylene surrounded by the jacket.

3. The suture of claim 2, wherein the yarns of ultrahigh molecular weight polyethylene coated with RGD peptide comprise at least 40% of the yarns in the suture.

4. The suture of claim 2, wherein the core comprises about 18% or greater of the total amount of yarns in the suture.

5. The suture of claim 2, wherein the jacket is formed of yarns of the ultrahigh molecular weight polyethylene braided with yarns of polyester.

6. The suture of claim 2, wherein the jacket is braided with trace yarns for suture identification.

7. The suture of claim 2, wherein the core is formed of at least three yarns of the ultrahigh molecular weight polyethylene twisted at three to six twists per inch.

8. The suture of claim 2, wherein the jacket is formed of yarns of ultrahigh molecular weight polyethylene braided with yarns of a bioabsorbable material comprising PLLA and collagen.

9. The suture of claim 2, wherein the jacket comprises at least eight yarns of the ultrahigh molecular weight polyethylene braided with six yarns of polyester and two yarns of nylon.

10. A suture assembly comprising the suture of claim 1 attached to a suture anchor.

11. A suture assembly comprising the suture of claim 1 attached to a needle.

12. The suture of claim 1, wherein the step of immersing the suture into a peptide solution further comprises allowing the peptides to react on the suture at 4°C.

13. The suture of claim 1, wherein:
in the step of soaking the suture into a buffer, an MES buffer is used and soaking takes place for at least 30 minutes;
in the acid hydrolysis step, the suture is dipped into an acid medium; and
the suture is immersed into the peptide solution to allow peptides to react on the suture at 4°C and to couple to the suture.

## Patentansprüche

1. Nahtmaterial mit einer Hülle, die mehrere umflochtene Garne aufweist, die aus ultrahochmolekularem Polyethylen bestehen, **dadurch gekennzeichnet, dass** das Nahtmaterial mit RGD-Peptid beschichtet ist, wobei das Nahtmaterial durch ein Verfahren beschichtet wird, das die folgenden Schritte aufweist:
Einweichen eines Nahtmaterials in einen MES-Puffer;
Entfernen des Nahtmaterials aus dem MES-Puffer, Durchführen einer Säurehydrolyse an dem Nahtmaterial und anschließend Wiedereinbringen des Nahtmaterials in den MES-Puffer;
Entfernen des Nahtmaterials aus dem MES-Puffer und Einbringen des Nahtmaterials in einen MES-Puffer, der EDC und Sulfo-NHS aufweist; und
Waschen des Nahtmaterials mit dem MES-Puffer, der EDC und Sulfo-NHS aufweist, und Eintauchen des Nahtmaterials in eine Peptidlösung, um eine Verbindung der Peptide mit dem Nahtmaterial zu ermöglichen, um das RGD-beschichtete Nahtmaterial zu bilden.

2. Nahtmaterial nach Anspruch 1, das ferner einen Kern mit Garnen aus einem ultrahochmolekularen Polyethylen aufweist, der von der Hülle umgeben ist.

3. Nahtmaterial nach Anspruch 2, wobei die mit RGD-Peptid beschichteten Garne aus ultrahochmolekularem Polyethylen mindestens 40% der Garne in dem Nahtmaterial umfassen.

4. Nahtmaterial nach Anspruch 2, wobei der Kern etwa 18% oder mehr der Gesamtmenge der Garne in dem Nahtmaterial umfasst.

5. Nahtmaterial nach Anspruch 2, wobei die Hülle aus Garnen aus dem ultrahochmolekularen Polyethylen besteht, die mit Garnen aus Polyester verflochten sind.

6. Nahtmaterial nach Anspruch 2, wobei die Hülle mit Beilauffäden zur Kennzeichnung des Nahtmaterials verflochten ist.

7. Nahtmaterial nach Anspruch 2, wobei der Kern aus mindestens drei Garnen aus dem ultrahochmolekularen Polyethylen gebildet wird, die mit drei bis sechs Drehungen pro Zoll gezwirnt sind.

8. Nahtmaterial nach Anspruch 2, wobei die Hülle aus Garnen aus ultrahochmolekularem Polyethylen gebildet wird, die mit Garnen aus einem bioabsorbierbaren Material verflochten sind, das PLLA und Kollagen aufweist.

9. Nahtmaterial nach Anspruch 2, wobei die Hülle mindestens acht Garne aus dem ultrahochmolekularen Polyethylen umfasst, die mit sechs Garnen aus Polyester und zwei Garnen aus Nylon verflochten sind.

10. Nahtmaterialeinheit, die das Nahtmaterial nach Anspruch 1 umfasst, das an einem Nahtanker befestigt ist.

11. Nahtmaterialeinheit, die das Nahtmaterial nach Anspruch 1 umfasst, das an einer Nadel befestigt ist.

12. Nahtmaterial nach Anspruch 1, wobei der Schritt zum Eintauchen des Nahtmaterials in eine Peptidlösung ferner beinhaltet, dass eine Reaktion der Peptide an dem Nahtmaterial bei 4°C ermöglicht wird.

13. Nahtmaterial nach Anspruch 1, wobei:
in dem Schritt zum Einweichen des Nahtmaterials in einen Puffer ein MES-Puffer verwendet wird und das Einweichen mindestens 30 Minuten lang erfolgt;
in dem Säurehydrolyseschritt das Nahtmaterial in ein Säuremedium eingetaucht wird; und
das Nahtmaterial in die Peptidlösung eingetaucht wird, um die Peptide bei 4°C an dem Nahtmaterial reagieren und an das Nahtmaterial binden zu lassen.

## Revendications

1. Suture comprenant une enveloppe comprenant une pluralité de fils tressés formés de polyéthylène à poids moléculaire ultra-élevé, **caractérisée en ce que** la suture est revêtue avec un peptide RGD, la suture étant revêtue par un procédé comprenant les étapes:
de trempage d'une suture dans un tampon MES;
d'enlèvement de la suture du tampon MES, de réalisation d'une hydrolyse acide sur la suture et de replacement par la suite de la suture dans le tampon MES;
d'enlèvement de la suture du tampon MES et de son placement dans un tampon MES comprenant EDC et sulfo-NHS; et
de lavage de la suture avec le tampon MES comprenant EDC et sulfo-NHS et d'immersion de la suture dans une solution de peptide pour permettre aux peptides de se coupler à la suture, pour former la suture revêtue de RGD.

2. Suture selon la revendication 1, comprenant en outre un coeur comprenant des fils d'un polyéthylène à poids moléculaire ultra-élevé entourés par l'enveloppe.

3. Suture selon la revendication 2, dans laquelle les fils de polyéthylène à poids moléculaire ultra-élevé revêtus avec un peptide RGD comprennent au moins 40% des fils dans la suture.

4. Suture selon la revendication 2, dans laquelle le coeur comprend environ 18% ou plus de la quantité totale de fils dans la suture.

5. Suture selon la revendication 2, dans laquelle l'enveloppe est formée de fils du polyéthylène à poids moléculaire ultra-élevé tressés avec des fils de polyester.

6. Suture selon la revendication 2, dans laquelle l'enveloppe est tressée avec des fils de trace pour une identification de la suture.

7. Suture selon la revendication 2, dans laquelle le coeur est formé d'au moins trois fils du polyéthylène à poids moléculaire ultra-élevé torsadés à trois à six torsades par pouce.

8. Suture selon la revendication 2, dans laquelle l'enveloppe est formée de fils de polyéthylène à poids moléculaire ultra-élevé tressés avec des fils d'un matériau bioabsorbable comprenant PLLA et du collagène.

9. Suture selon la revendication 2, dans laquelle l'enveloppe comprend au moins huit fils du polyéthylène à poids moléculaire ultra-élevé tressés avec six fils de polyesters et deux fils de nylon.

10. Assemblage de suture comprenant la suture selon la revendication 1 attachée à un ancrage de suture.

11. Assemblage de suture comprenant la suture selon la revendication 1 attachée à une aiguille.

12. Suture selon la revendication 1, dans laquelle l'étape d'immersion de la suture dans une solution de peptide comprend en outre l'étape consistant à permettre aux peptides de réagir sur la suture à 4°C.

13. Suture selon la revendication 1, dans laquelle:
dans l'étape de trempage de la suture dans un tampon, un tampon MES est utilisé et le trempage a lieu pendant au moins 30 minutes;
dans l'étape d'hydrolyse acide, la suture est plongée dans un milieu acide; et
la suture est immergée dans la solution de peptide pour permettre aux peptides de réagir sur la suture à 4°C et de se coupler à la suture.
